# EUROPEAN PATENT APPLICATION

(11) **EP 4 792 786 A1**
(43) Date of publication of application: **19.08.2026**
(21) Application number: 26157165.7
(22) Date of filing: 09.02.2026
(51) Int. Cl.: A61F 5/56

(54) **MANDIBULAR REPOSITIONING MOUTH DEVICE**

(30) Priority: 17.02.2025 BR 102025003116
(71) Applicant: Kozokic, Jovana, 6855 Stabio (CH)
(72) Inventor: Kozokic, Jovana, 6855 Stabio (CH)
(74) Representative: Ellberg, Nils

(57) **Abstract**

The present invention relates to a mandibular REPOSITIONING mouthpiece comprising an upper molded portion and a lower molded portion separated from each other. The upper molded portion of the device comprises a right protrusion and a left protrusion, each protrusion being configured for the entry of a protrusion piston. The lower molded portion of the device comprises two wings configured to move the mandible forward. In addition, the lower molded portion also comprises holes in its upper surface, in which the holes are configured to fit ascending plates configured to modify the vertical dimension of the mandible.

## Description

### TECHNICAL FIELD

The present invention relates to an oral device for mandibular reposition to aid in the treatment and relief of the symptoms of obstructive apnea syndrome.

### DESCRIPTION OF THE STATE OF THE ART

It is well known that obstructive sleep apnea causes the throat or upper airway to close repeatedly, so that breathing can become abnormally slow and shallow, or can stop suddenly (usually more than 10 seconds) and then return to normal. In severe cases of obstructive sleep apnea, repeated attacks of choking and snoring occur during the night, while during the day there is excessive sleepiness or episodes of involuntary napping.

Specifically, during sleep, the muscles of the pharynx relax and, if the circumference of the neck increases or the muscles are excessively relaxed, the airways narrow, causing the person to stop breathing. As a result, oxygen saturation decreases and the nervous system is activated, triggering two reactions: the person struggles to breathe (fighting to regain their breath) and there is an arousal or awakening from deep sleep. This cycle tends to repeat itself throughout the night.

During arousal, the body wakes up and muscle tone is restored, and the person begins to breathe. This sleep fragmentation and oxygen desaturation leads to excessive daytime sleepiness.

This disorder is associated with reduced quality of life, increased mortality and cardiovascular disease, cerebrovascular events, diabetes, and cognitive impairment.

Another occurrence is hypopnea, in which there is a reduction in airflow between 30% and 90% for at least 10 seconds during sleep. Obstructive Sleep Apnea and Hypopnea Syndrome (OSAHS) is classified according to its severity in terms of the Hourly Apnea/Hypopnea Index (AHI), which is the number of times the event occurs per hour of sleep, i.e. AHI<5= NORMAL; AHI between 5 and 15= mild apnea; AHI between 15 and 30= moderate apnea; AHI>30= severe apnea.

Several removable oral devices or appliances developed by dentists are already known to help relieve obstructive sleep apnea (and snoring) in patients with mild to moderate apnea. In general, the treatment for severe cases of obstructive sleep apnea (OSAS) is CPAP (Continuous Positive Airway Pressure).

These devices, worn exclusively during sleep, help to keep the airways clear. Most appliances are made up of two pieces of plastic, molded to fit the upper and lower teeth. These pieces are interconnected and designed to bring the jaw forward, so that the base of the tongue moves away from the back wall of the larynx, thus preventing the tongue from moving backwards and blocking the throat.

For example, document US 9,204,991 B1 describes a mandibular advancement device comprising a maxillary tray for receiving and retaining maxillary teeth and a mandibular tray for receiving and retaining mandibular teeth. The mandibular tray comprises protruding members/points that contact an external surface of the maxillary tray to advance the mandibular protrusive position. The mandibular tray comprises raised occlusal surfaces that can be raised or lowered in height to further increase the airway space.

Similarly, document US2020069457 describes a set of mandibular advancement splints for treating sleep apnea. The maxillary splint and mandibular splint comprise a maxillary abutment assembly and a complementary mandibular abutment assembly that cooperate to provide the desired mandibular advancement. The maxillary and mandibular splint assembly comprises a mandibular abutment assembly and adjustment spacers. When the mandibular abutment assembly and spacers touch each other, this produces the desired mandibular protrusion.

However, these documents focus only on altering mandibular protrusion and do not consider the vertical dimension for the purpose of relieving apnea symptoms.

The evaluation of obstructive sleep apnea should not be limited solely to mandibular protrusion; rather, the vertical dimension must also be considered. Mandibular repositioning, rather than mere advancement, plays a crucial role in treatment. One of the key reasons for this approach is that increasing the vertical dimension provides additional space for the tongue, potentially improving airway patency.

### OBJECTIVES OF THE INVENTION

One of the objectives of the present invention is to provide an oral device for mandibular advancement that helps relieve the symptoms of obstructive sleep apnea, keeping the airways clear and improving the quality of sleep and reducing the negative effects on the health of those who suffer from apnea.

### BRIEF DESCRIPTION OF THE INVENTION

The present invention proposes a solution to the drawbacks of the previous technique by means of a mandibular advancement mouthpiece comprising an upper molded portion and a lower molded portion separated from each other.

The upper molded portion of the device comprises a right protrusion and a left protrusion, each protrusion being configured for the entry of a protrusion piston. In addition, each protrusion piston is fitted with at least one protrusion disk.

The lower molded portion of the device comprises two wings configured to move the mandible forward. In addition, the lower molded portion also comprises holes in its upper surface, where the holes are configured to fit ascending plates configured to modify the vertical dimension of the mandible.

### BRIEF DESCRIPTION OF THE FIGS.

The present invention will be described in more detail below on the basis of an example of execution shown in the drawings. The Figures show:
Fig. 1a illustrates a frontal view of one type of mandibular advancement device.
Fig. 1b illustrates a left side view of the device in Fig. 1a.
Fig. 1c illustrates a right-side view of the device in Fig. 1a.
Fig. 2a illustrates a frontal view of a modality of the upper and lower molded sections of the mandibular advancement mouthpiece joined together.
Fig. 2b illustrates a left side view of the device in Fig. 2a.
Fig. 2c illustrates a right-hand side view of the device in Fig. 2a.
Fig. 3 illustrates a protrusion piston with protrusion discs.
Fig. 4 illustrates the protrusion piston with protrusion discs from Fig. 3 fitted into a protrusion of the upper molded portion.
Fig. 5 illustrates an upper molded portion with both protrusion pistons with protrusion discs fitted into the protrusions.
Fig. 6a illustrates a top view of a modality of the lower molded portion with holes for fitting.
Fig. 6b illustrates a bottom view of the molded portion of Fig. 6a.
Fig. 7 illustrates an ascending plate modality with plug-in pins.
Fig. 8 illustrates a bottom view of the lower molded portion of Fig. 6a in the configuration with the ascending plates mounted.
Fig. 9 illustrates a top view of the lower molded portion of Fig. 6a in a configuration with only one riser plate mounted.

### DETAILED DESCRIPTION OF THE FIGS.

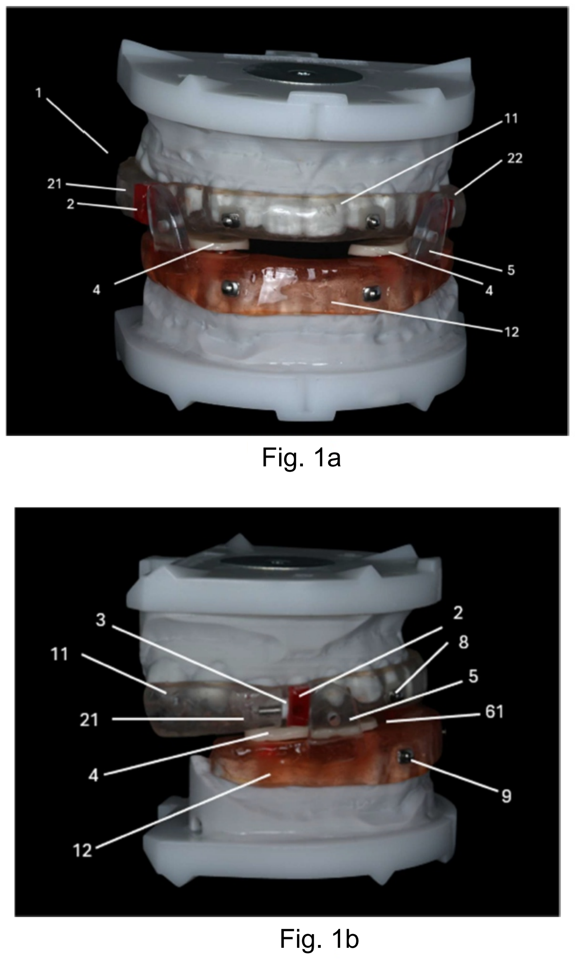

Figs. 1a-c represent front and side views of an exemplary embodiment of the present invention. The mandibular advancement mouthpiece 1 according to the invention comprises an upper molded portion 11 and a lower molded portion 12 separated from each other.

The upper and lower molded portions 11, 12 of device 1, as shown in Figs. 1a-c, are portions molded to each patient's dental arch. The device must cover all the teeth so as not to risk extrusion of the tooth that is not covered. If there are wisdom teeth in the dental arch, even these teeth need to be covered. To produce the molded portions 11, 12, the patient's teeth are scanned, and the patient's maximum mandibular protrusion and maximum vertical dimension are measured. The patient's bite at maximum intercuspation and at 50% of mandibular protrusion is also recorded for the upper and lower molded portions 11, 12.

In the illustrated embodiment, the invention is also equipped with two protrusion pistons 2, two protrusion discs 3 for each protrusion piston 2 and two ascending plates 4. These elements can be used in a standard configuration for all patients and do not require customization.

In the illustrated embodiment, the upper molded portion 11 has a right protrusion 21 and a left protrusion 22 on each of its posterior outer sides. Each of the right and left protrusions 21, 22 is configured to receive a protrusion piston 2. The protrusion pistons 2 are each configured to receive at least one protrusion disk 3. Protrusion pistons 2 having protrusion disks 3 are fitted into protrusions 21, 22 so that they are attached parallel to the upper molded portion 11.

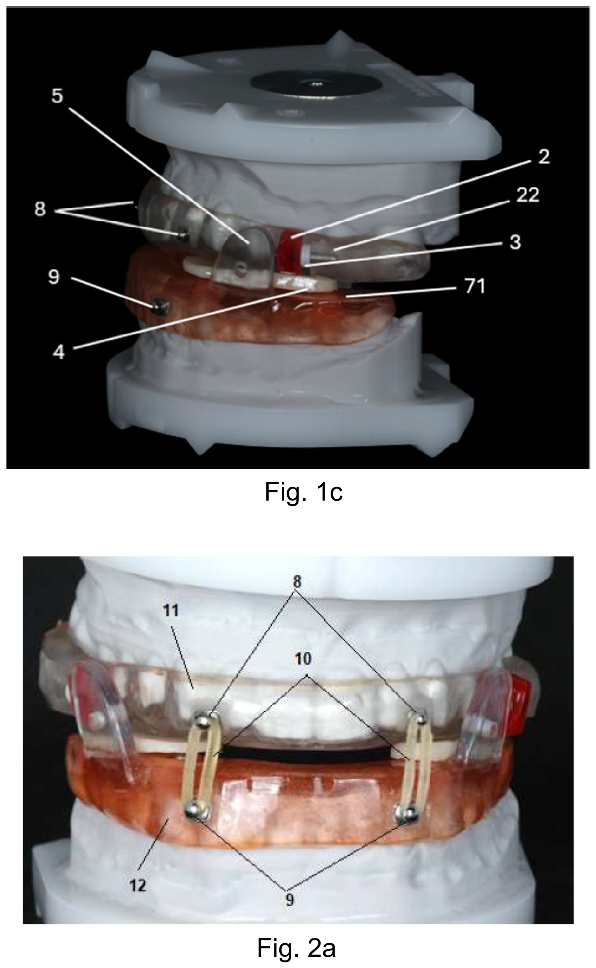

The lower molded portion 12 of the device 1 has two wings 5. Each of the two wings 5 is located on the outer right and outer left sides of the lower molded portion 12. The wings 5 are designed in such a way that the protrusion pistons 2 of the upper molded portion 11 push them displacing the mandible forward. In addition, as each protrusion disk 3 is added to each protrusion piston 2, the mandibular protrusion increases, thus shifting the mandible forward. The lower molded portion 12 also has a first ascending plate 4 fitted to its upper right surface 61 as shown in Fig. 1b, and a second ascending plate 4 located on its upper left surface 71 as shown in Fig. 1c, configured to modify the vertical dimension of the mandible.

Usually, for maximum efficiency of the treatment carried out by the device of the present invention, the protrusion needs to be increased by 1 mm per month until the patient does not feel well when sleeping or claims that he does not snore. The protrusion is increased by adding additional protrusion discs 3 to the protrusion pistons 2.

Optionally, the upper molded portion 11 and the lower molded portion 12 also have at least two upper hooks 8 and two lower hooks 9 attached to each of them. In a preferred embodiment of the invention, hooks 8 of the upper molded portion are aligned vertically with hooks 9 of the lower molded portion.

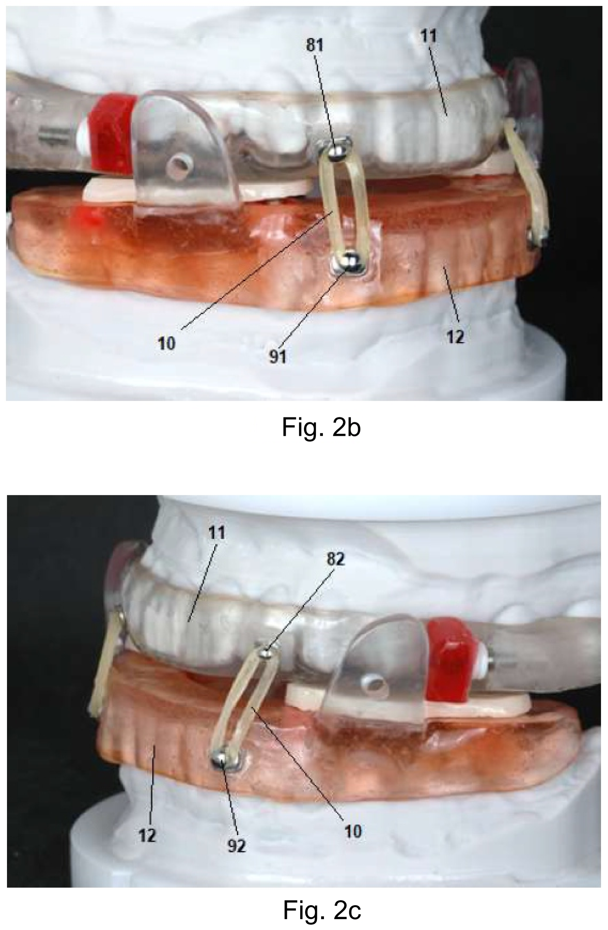

Figs. 2a-c illustrate an optional modality of the present invention, with two upper hooks 8, being an upper right hook 81 and an upper left hook 82 and two lower hooks 9, being a lower right hook 91 and a lower left hook 92. The upper right hook 81 of the patient's upper molded portion 11 is especially located so as to be vertically aligned with the lower right hook 91 of the patient's lower molded portion 12 to receive a first elastic 10. Likewise, the upper left hook 82 of the patient's upper molded portion 11 is especially located so as to be vertically aligned with the lower left hook 92 of the patient's lower molded portion 12 to receive a second elastic 10 that joins the two upper and lower molded portions 11, 12. The elastics 10 thus ensure the stability of the two upper and lower molded portions 11, 12 together.

In a preferred embodiment of the invention, the minimum vertical dimension of each of the upper and lower molded portions 11, 12 is at least 1,5 mm.

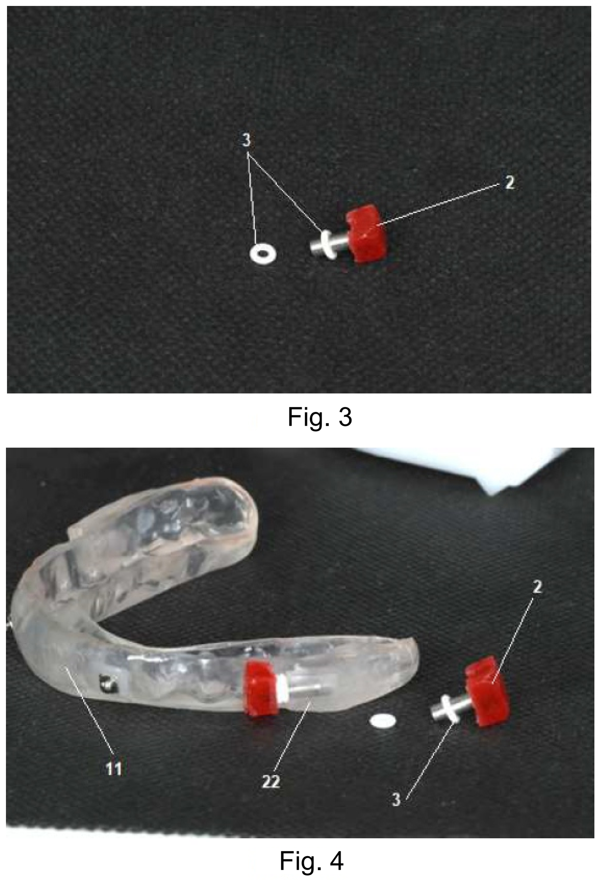

Fig. 3 illustrates one way of the protrusion piston 2 in the disassembled configuration of device 1. The protrusion piston 2 can fit as many discs 3 as necessary in order to achieve the perfect protrusion. The protrusion disk 3 is preferably 1 mm thick.

Fig. 4 illustrates a partially assembled configuration of device 1, in which a protrusion piston 2 containing two protrusion discs 3 is fitted into the left lateral protrusion 22 outside the upper molded portion 11. The fitting of the protrusion pistons 2 into the lateral protrusions is carried out, preferably by pressure. Alternatively, other fittings can be used, such as by thread or similar.

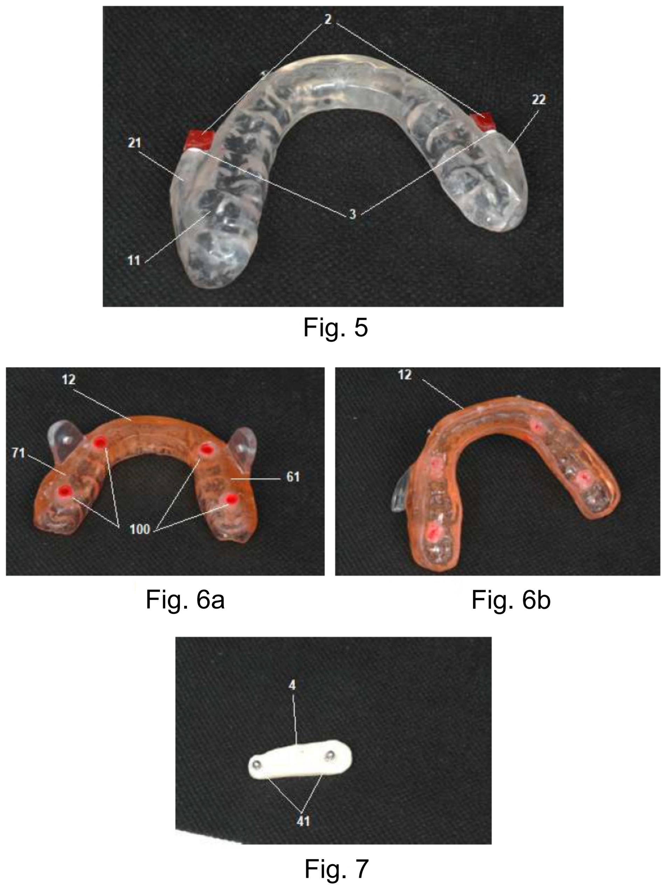

The fully assembled configuration of the upper impression portion 11 is illustrated in Fig. 5 with its fitting face to the dental arch facing downwards. In Fig. 5, the two protrusion pistons 2 containing protrusion disks 3 are fitted into the external right lateral protrusion 21 and the external left lateral protrusion 22 of the portion.

Figs. 6a and 6b represent an exemplary modality of the lower molded portion 12.

The lower molded portion 12 contains at least one, preferably two holes 100 on each side of its upper surface. The holes 100 on the upper right surface 61 are configured to receive the right ascending plate 4, and the two holes on the upper left surface 71 are configured to receive the left ascending plate 4. In a bottom view of the lower molded portion 12 it can be seen that the holes 100 are non-passing, so that the pins to be engaged in them do not touch the patient's teeth.

Fig. 7 illustrates an exemplary riser plate 4 comprising two pins 41 configured to engage with the holes 100 of a respective upper surface 61, 71 of the lower molded portion 12. In the preferred embodiment, the two pins 41 are each located at one end of the riser plate 4 and promote engagement by pressure via a spherical tip. By inserting the spherical tip of the pin 41 into the respective hole 100, the ascending plate 4 remains firmly in position. The mandibular advancement mouthpiece 1 is compatible with multiple ascending plate configurations 4, for example with heights of 1 mm, 2 mm, 3 mm, 4 mm, 5 mm, 6 mm, and 7 mm. In addition, in the preferred mode, the rising plates 4 have a configuration length that arrives from distal of canine up to the distal of the first molar of a patient and a width of, approximately 1 cm. In some cases, it may be necessary to modify the vertical dimension of the device for the best patient outcome during treatment. This is done by changing the ascending plate 4 to a plate with a higher height.

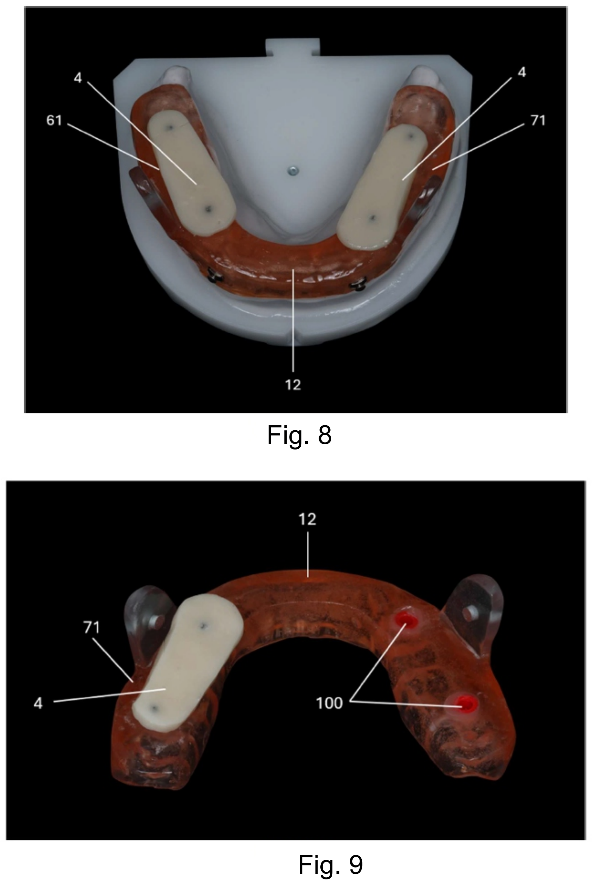

Figs. 8 and 9 illustrate, respectively, a fully assembled configuration with two rising plates 4 fitted to the upper surfaces 61, 71 of the lower molded portion 12 and a partially assembled configuration with only one rising plate 4 fitted to the upper left surface 71. The right and left ascending plates 4 are configured to increase the vertical dimension of the mandible according to the height of the ascending plates 4, which can vary, preferably from 1 mm up to the maximum vertical dimension opening from a patient that was measured from the beginning of the treatment.

An example of a preferred embodiment having been described, it should be understood that the scope of the present invention covers other possible variations and is limited only by the content of the appended claims, including possible equivalents.

### List of Reference Numerals:

1 - oral device for mandibular advancement
11 - upper molded portion
12 - lower molded portion
2 - protrusion pistons
21 - right overhang, right side overhang
22 - left overhang, left side overhang
3 - protrusion disks
4 - plate ascending
41 - pins
5 - wings
61 - upper right surface
71 - upper left surface
8 - top hooks
81 - upper right hook
82 - upper left hook
9 - bottom hooks
91 - lower right hook
92 - lower left hook
10 - rubber bands
100 - holes

## Claims

1. Oral device for mandibular advancement (1), **characterized by the** fact that it comprises an upper molded portion (11) and a lower molded portion (12) separated from each other; wherein the upper molded portion (11) of the device (1) comprises a right protrusion and a left protrusion (21, 22), each protrusion being configured for entry d and a protrusion piston (2), wherein at least one protrusion disk (3) is arranged on each protrusion piston (2); wherein the lower molded portion (12) of the device (1) comprises a right wing and a left wing (5) configured to be pushed by the protrusion pistons (2), and wherein the lower molded portion (12) comprises yet holes (100) on the upper surface (61, 71) thereof, wherein the holes (100) are configured to fit plates ascending (4) configured to modify the vertical dimension of the mandible.

2. Mouth device for mandibular advancement (1), according to claim 1, **characterized by the** fact that each of the upper and lower molded portions (11, 12) has at least two upper hooks (8) and two lower hooks (9) fixed to them.

3. Mouth device for mandibular advancement (1), according to claim 2, **characterized by the** fact that the upper hooks (8) and the lower hooks (9) are configured to receive elastics (10) capable of joining the upper molded portion (11) to the lower molded portion (12).

4. Oral device for mandibular advancement (1), according to claim 1, **characterized in that** the at least one protrusion disc (3) has a thickness of 1 mm.

5. Oral device for mandibular advancement (1), according to claim 1, **characterized by the** fact that the ascending plates (4) have a height of 1 mm to the maximum vertical dimension of a patient that was measured at the beginning of the treatment.

6. Oral device for mandibular advancement (1), according to claim 1, **characterized by the** fact that the ascending plates (4) have a length from distal of canine up to the distal of the first molar of a patient and a width of 1 cm each.

7. Oral device for mandibular advancement (1), according to claim 1, **characterized in that** the minimum vertical dimension in each upper and lower molded portion (11, 12) is at least 1,5 mm.

8. Mouth device for mandibular advancement (1), according to claim 1, **characterized in that** each protrusion piston (2) can receive as many protrusion discs (3) as needed in order to achieve the maximum protrusion measured at the beginning of the treatment.
